# EUROPEAN PATENT SPECIFICATION

(11) **EP 4 076 172 B1**
(45) Date of publication and mention of the grant of the patent: **20.05.2026**
(21) Application number: 20833851.7
(22) Date of filing: 17.12.2020
(51) Int. Cl.: A61B 5/06, A61B 5/291

(54) **IMPLANTABLE ARRAY WITH A REFERENCE STRUCTURE AND METHOD OF MANUFACTURING THE SAME**
IMPLANTIERBARES ARRAY MIT REFERENZSTRUKTUR UND VERFAHREN ZU DESSEN HERSTELLUNG
RÉSEAU IMPLANTABLE AVEC UNE STRUCTURE DE RÉFÉRENCE ET SON PROCÉDÉ DE FABRICATION

(30) Priority: 19.12.2019 EP 19218170
(43) Date of publication of application: 26.10.2022
(73) Proprietor: CorTec GmbH, 79108 Freiburg (DE)
(72) Inventor: ERHARDT, Johannes, 79415 Bad Bellingen (DE); SCHÜTTLER, Martin, 79312 Emmendingen (DE); STIEGLITZ, Thomas, 79110 Freiburg (DE); BOCK, Michael, 69126 Heidelberg (DE)
(74) Representative: 2s-ip Schramm Schneider Bertagnoll Patent- und Rechtsanwälte Part mbB
(86) International application number: PCT/EP2020/086881
(87) International publication number: WO 2021/123058

(56) References cited:
- US-A1- 2015 297 316
- US-A1- 2016 120 457
- US-A1- 2018 369 572
- ERHARDT J B ET AL: "Precise localization of silicone-based intercranial planar electrodes in magnetic resonance imaging", 2017 39TH ANNUAL INTERNATIONAL CONFERENCE OF THE IEEE ENGINEERING IN MEDICINE AND BIOLOGY SOCIETY (EMBC), IEEE, 11 July 2017 (2017-07-11), pages 513 - 516, XP033152099, DOI: 10.1109/EMBC.2017.8036874
- YANG A I ET AL: "Localization of dense intracranial electrode arrays using magnetic resonance imaging", NEUROIMAGE, ELSEVIER, AMSTERDAM, NL, vol. 63, no. 1, 15 October 2012 (2012-10-15), pages 157 - 165, XP002715902, ISSN: 1053-8119, [retrieved on 20120630], DOI: 10.1016/J.NEUROIMAGE.2012.06.039

## Description

The present invention relates to an implantable electrode array, with a reference structure, and to a method of manufacturing the implantable electrode array, , with a reference structure which is visible in magnetic resonance images (MRI) when implanted for example in a human or animal body.

Such an array is implanted on the surface of the brain e.g., for pre-surgical assessment of cortical electrical activity in patients with epilepsy.

Hereby, MRI is used to localize the electrode contacts of the implanted array with respect to the anatomy of the patient. However, metal components or other good electric current conductive materials like carbon and electric conductive polymers may cause MRI artifacts that compromise the results especially in the direct vicinity of the implants. Thin-film implants which may feature 100x less metal thickness may mitigate these artifacts, but thin-film implants produce inconspicuous MRI signal voids in many clinical MRI sequences, and are therefore not suitable for localizing the electrode with MRI.

On the other hand, implants having very small metal contacts, very thin metal layers, or no metal at all may not become visible in clinical MRI at all.

But imperatively, physicians need to know the implant position, and the value of intracranial EEG increases with the precision of electrode localization.

ERHARDT J B ET AL, "Precise localization of silicone-based intercranial planar electrodes in magnetic resonance imaging", 2017 39TH ANNUAL INTERNATIONAL CONFERENCE OF THE IEEE ENGINEERING IN MEDICINE AND BIOLOGY SOCIETY (EMBC), IEEE, (20170711), doi:10.1109/EMBC.2017.8036874, pages 513 - 516, XP033152099, relates to an implantable electrode array with the features of the preamble of claim 1.

YANG A I ET AL, in NEUROIMAGE, ELSEVIER, AMSTERDAM, NL, vol. 63, no. 1, doi:10.1016/J.NEUROIMAGE.2012.06.039, ISSN 1053-8119, (20121015), pages 157 - 165, (20120630), XP002715902 relates to localization of dense intracranial electrode arrays using magnetic resonance imaging.

US 2018/369572 A1 relates to a device with the features of the preamble of claim 1.

US 2015/297316 A1 relates to tissue markers and uses thereof, e.g., to mark a target tissue site or to produce a cell scaffold.

The present invention is based on the problem to provide an implantable electrode array for MRI-based localization of its standard or micro-sized planar electrode contacts, and a method of manufacturing such electrode arrays which can be precisely localized in MRI.

This problem is solved by the implantable electrode array, and the method of manufacturing the implantable electrode array, according to the respective independent claim. Further aspects of the invention are defined in the dependent claims.

Advantageous embodiments of the invention may comprise the following features.

The patterns may comprise a material having a magnetic susceptibility which is higher than the magnetic susceptibility of the anatomic tissue surrounding the electrode array when placed in the human or animal body.

Each pattern may be isolated and spaced apart from each other pattern.

The structure may form a grid over the predefined portion of the electrode array.

The predefined form may be a cross-like form.

The predefined portion may comprise at least one of
- a top surface,
- a rear surface,
- an edge,
- a corner.

At least three patterns may be aligned along a straight first line, and at least three patterns may be aligned along a straight second line, the second line being perpendicular to the first line.

The implantable electrode array may comprise at least one layer of a polymer, in particular selected from the group comprising silicone rubber, polyurethane, polyimide, epoxy, liquid crystal polymer, and parylene.

The contacts may form a first grid with a first grid constant, and the patterns may form a second grid with the same grid constant as the metal contacts.

The polymer may be selected from the group comprising silicone rubber, polyurethane, polyimide, epoxy, liquid crystal polymer, and parylene.

The conductive layer may be selected from the group comprising metal, conductive polymer, carbon.

The material may have a magnetic susceptibility which is higher than the magnetic susceptibility of the anatomic tissue surrounding the electrode array when placed in the human or animal body.

The invention and embodiments thereof will be described below in further detail in connection with the drawing.
- Fig. 1: illustrates the implantable electrode array according to an embodiment of the invention,
- Fig. 2: illustrates distances in the implantable electrode array according to an embodiment of the invention,
- Fig. 3: illustrates sectional view of the implantable electrode array according to an embodiment of the invention,
- Fig. 4: illustrates the implantable electrode array according to a further embodiment of the invention.

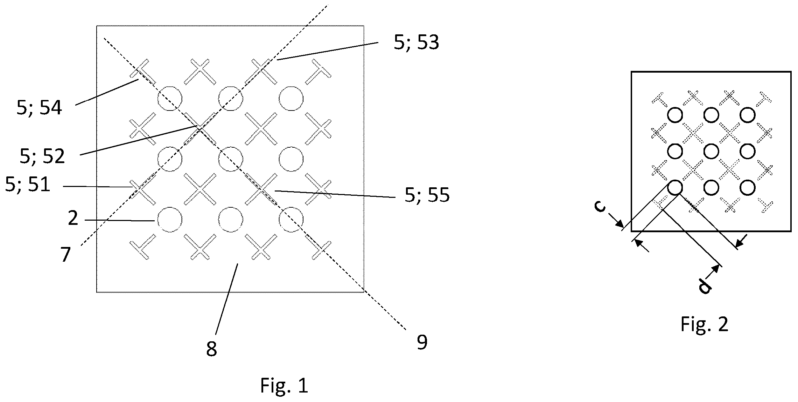

Fig. 1 and Fig. 2 illustrate an implantable electrode array according to an embodiment of the invention. The implantable electrode array comprises at least one substrate layer 1 made from a biocompatible polymer. The polymer can be selected from the group comprising silicone rubber, polyurethane, polyimide, epoxy, liquid crystal polymer, and parylene.

The implantable electrode array is flexible such that it can adapt its form to the form of the location in the cortex of a human or animal where it is placed. The electrode array comprises sensor contacts 2 of diameter c on its surface 8. The electrode contacts 2 may be located in apertures 6 in the polymer 1 of a diameter smaller than diameter c.

The electrode array further comprises a structure 5 for referencing predefined distinct points 2, e.g., the contacts 2, of the implantable electrode array in MRI. The structure 5 is arranged on the implantable electrode array, i.e., on the surface 8 or under the surface 8, and comprises single patterns 51, 52, 53.

Each pattern 51, 52, 53 comprises particles (i.e., nanoparticles, NPs) with a material producing a large magnetic susceptibility difference with respect to the surroundings, that is the tissue of the patient where the electrode array is to be placed.

This difference in magnetic susceptibility is linked with a difference in the respective effective transverse relaxation times (T2*), e.g., a very short T2* for the material with a high magnetic susceptibility, and a longer T2* for the surrounding human tissue, having a smaller magnetic susceptibility, such that the MRI signal in and near the pattern is lost, and thus a contrast to the surrounding material is established with the result that the pattern becomes visible in a magnetic resonance image of the electrode array.

The material having a very short T2* for the patterns can be selected from the group comprising, e.g., iron, iron oxide and oxides of rare earths.

The reference structure 5 forms a grid over a predefined portion of the electrode array; each pattern 51, 52, 53, 54, 55 is in a predefined spatial relationship, e.g., distance d, to one of the metal contacts 2 to be referenced.

As can be seen from the figure, each pattern 51, 52, 53, 54, 55 is isolated and spaced apart from each other pattern. The patterns may have cross-like form.

The patterns 51, 52, 53, 54, 55 may be equidistant to each other. At least two patterns 51, 52, 53, 54, 55 may be aligned along a straight first line 7. Moreover, at least two patterns 52, 54, 55 may be aligned along a straight second line 9, the second line 9 being perpendicular to the first line 7. In this example, the patterns form a regular 2-dimensional grid over the surface 8 of the electrode array, as indicated in Fig. 2.

However, other forms for the structure 5 are also possible.

As can be seen, the metal contacts form a first grid with a first grid constant, and the patterns form a second grid with the same grid constant as the metal contacts.

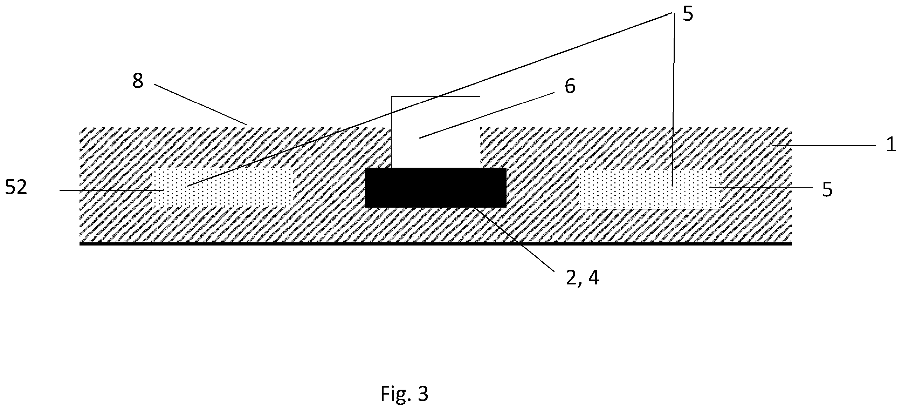

In sectional view of the implantable electrode array according to Fig. 3, the reference structure 5 is formed under the surface 8 of the substrate 1, i.e., each pattern 51, 52, 53, 54, 55 of the reference structure 5 is surrounded by the substrate 1.

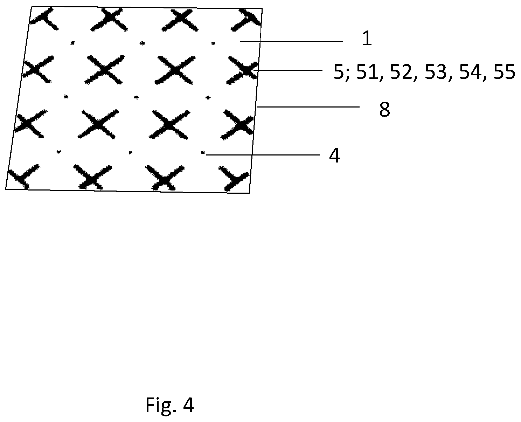

Typical diameters c of the electrode contacts 2 are 4 mm. The apertures 6 may have a diameter of 2.3 mm. Typical electrode arrays may comprise e.g. 3x3 electrode contacts or 3x6 electrode contacts, depending on the intended application. A typical spacing between two electrode contacts 2 (interelectrode spacing) may be 10 mm. Microelectrode arrays, according to a further embodiment of the invention as illustrated in Fig. 4, may have contacts 4 with diameters of about 0.3 mm.

According to a particular variant, the patterns 51, 52, 53, 54, 55 of reference structure 5 may comprise silicone rubber doped with iron oxide (e.g. Fe₂O₃) particles. Herein, iron oxide is selected for its good ferromagnetic properties and its very good biocompatibility.

The predefined points 2 which are referenced by the patterns 51, 52, 53, 54, 55 are electrical contacts 2, i.e., sensor contacts; selected from metal, conductive polymer, carbon on the implantable electrode array.

As mentioned above, the structure 5 may form a grid over a predefined portion of the electrode array. The predefined portion may comprise or be at least one of the top surface 8 (i.e., the side where the contacts are), the rear surface (i.e., the side where no contact is), an edge, and a corner of the electrode array, or be in the interior of the electrode array.

Table 1 gives typical, non-limiting dimensions of the implantable electrode arrays with reference structures 5.

**Table 1**

| | | |
|---|---|---|
| Electrode diameter in mm | 0.3 | 4.0 |
| Electrode material | Pt/Ir | MP35N |
| Total sample thickness in mm | 0.29 | 0.27 |
| Reference structure thickness in mm | 0.29 | 0.27 |
| Reference structure design width in mm | 0.50 | 0.50 |

In the following, a manufacturing process of an implantable electrode array with a reference structure 5 is described.

The manufacturing is based on a layer-by-layer deposition method, starting with a mechanical carrier, typically a ceramic substrate: The method comprises the following steps:
a. A layer of preferably some 10µm to a few 100µm thick liquid silicone rubber is deposited. The silicone is cured.
b. A metal foil of preferably some µm to a few 10µm thickness is laminated to the silicone. The metal is patterned with a laser, removing all metal but the electrode contacts, interconnection tracks and weld pads.
c. A second layer of silicone is deposited of preferably some 10µm to a few 100µm thickness. The silicone is cured.
d. Trenches are cut into the silicone using a laser. Theses trenches define the shape of the MRI reference structures. The depth of the trenches defined the layer height of the reference structures.
e. The trenches are filled with silicone rubber containing superparamagnetic nanoparticles. The filling of the trenches is cured.
f. A covering layer of silicone rubber of preferably some 10µm to a few 100µm is deposited and cured.
g. Metal contacts are exposed by removing silicone with a laser.
h. The outline of the electrode array is defined by cutting through all deposited layers with a laser.
i. The electrode array is separated from the mechanical carrier.

Instead of silicone rubber, any flexible polymer can be used, in particular one from the group comprising silicone rubber, polyurethane, polyimide, epoxy, liquid crystal polymer, and parylene.

The electrode array as described above is particularly well suited for being imaged with MRI.

Advantageously, the reference structures as described above can be freely shaped in all dimensions, and the NP concentration can be varied. As a result, the implant thickness could be as little as 50 µm and thus feature high flexibility. Since these reference structures do not alter the outside silicone slab shape, the handling of these implants is identical to that of conventional implants. Due to their distinct contrast to the surroundings, the reference structures may be especially useful for electrode contact placement in sulci. Since the patterns of the reference structure can be placed on the complete surface of the electrode array like a grid, they can reference the electrode contacts in situ even if the electrode is strongly bent or curved.

The invention is defined by the appended claims.

## Claims

1. An implantable electrode array suitable for being placed in anatomic tissue of a human or animal body, comprising
a structure (5) for referencing predefined distinct points (2) of the implantable electrode array in magnetic resonance images, the structure (5) being arranged in a predefined portion of the implantable electrode array, the structure (5) comprising:
a plurality of patterns (51, 52, 53, 54, 55), each pattern (51, 52, 53, 54, 55) having a predefined form and comprising a material having a magnetic susceptibility which is different from the magnetic susceptibility of the anatomic tissue surrounding the array when placed in the human or animal body,
each pattern (51, 52, 53, 54, 55) being in a predefined spatial relationship with one of the predefined distinct points (2), the predefined distinct points being electrode contacts,
wherein
the material is a polymer, in particular selected from the group comprising silicone rubber, polyurethane, polyimide, epoxy, liquid crystal polymer, and parylene,
**characterized in that**
the material comprises particles with a material selected from the group comprising iron, iron oxide, oxides of rare earths, and pyrolytic carbon material,
the electrode contacts form a first grid with a first grid constant and the patterns (51, 52, 53, 54, 55) form a second grid with the same grid constant as the one of the electrode contacts.

2. The implantable electrode array of claim 1, wherein
the patterns (51, 52, 53, 54, 55) comprise a material having a magnetic susceptibility which is higher than the magnetic susceptibility of the anatomic tissue surrounding the electrode array when placed in the human or animal body.

3. The implantable electrode array of one of the preceding claims, wherein
each pattern (51, 52, 53, 54, 55) is isolated and spaced apart from each other pattern (51, 52, 53, 54, 55).

4. The implantable electrode array of one of the preceding claims, wherein
the structure (5) forms a grid over the predefined portion of the electrode array.

5. The implantable electrode array of one of the preceding claims, wherein
the predefined portion comprises at least one of
- a top surface (8),
- a rear surface,
- an edge,
- a corner.

6. The implantable electrode array of one of the preceding claims, wherein
at least three patterns (51, 52, 53) are aligned along a straight first line, and at least three patterns (52, 54, 55) are aligned along a straight second line, the second line being perpendicular to the first line.

7. The implantable electrode array of one of the preceding claims, wherein
the implantable electrode array comprises at least one layer of a polymer, in particular selected from the group comprising silicone rubber, polyurethane, polyimide, epoxy, liquid crystal polymer, and parylene.

8. Method of manufacturing an implantable electrode array, suitable for being placed in anatomic tissue of a human or animal body, according to one of the preceding claims, comprising the following steps:
a. A first layer of a polymer is deposited on a mechanical carrier;
b. The first polymer layer is cured;
c. A conductive layer is deposited onto the first polymer layer;
d. The conductive layer is patterned with a laser, thus forming electrode contacts (2), interconnection tracks and weld pads;
e. A second layer of polymer is deposited;
f. The second layer of polymer is cured;
g. Trenches are cut into the polymer layer using a laser, the trenches defining a MRI reference structure (5);
h. The trenches are filled with a polymer comprising a material having a magnetic susceptibility which is different from the magnetic susceptibility of the anatomic tissue surrounding the electrode array when placed in the human or animal body; the polymer being in particular selected from the group comprising silicone rubber, polyurethane, polyimide, epoxy, liquid crystal polymer, and parylene;
the polymer comprising particles with a material selected from the group comprising iron, iron oxide, oxides of rare earths, and pyrolytic carbon material;
i. The filling of the trenches is cured;
j. A covering layer of polymer is deposited and cured;
k. Electrical contacts are exposed by removing the covering layer with a laser;
l. The outline of the electrode array is defined by cutting through all deposited layers with a laser;
m. The electrode array is separated from the mechanical carrier.

9. The method of the preceding claim wherein
the polymer is selected from the group comprising silicone rubber, polyurethane, polyimide, epoxy, liquid crystal polymer, and parylene.

10. The method of the preceding claim wherein the conductive layer is selected from the group comprising metal, conductive polymer, carbon.

11. The method of one of claims 9 and 10, wherein the material has a magnetic susceptibility which is higher than the magnetic susceptibility of the anatomic tissue surrounding the electrode array when placed in the human or animal body.

## Patentansprüche

1. Implantierbare Elektrodenanordnung, die zum Einbringen in anatomisches Gewebe eines menschlichen oder tierischen Körpers geeignet ist, umfassend
eine Struktur (5) zum Referenzieren vordefinierter unterschiedlicher Punkte (2) der implantierbaren Elektrodenanordnung in Magnetresonanzbildern, wobei die Struktur (5) in einem vordefinierten Abschnitt der implantierbaren Elektrodenanordnung angeordnet ist, die Struktur (5) umfassend:
eine Vielzahl von Mustern (51, 52, 53, 54, 55), wobei jedes Muster (51, 52, 53, 54, 55) eine vordefinierte Form aufweist und ein Material umfasst, das eine magnetische Suszeptibilität aufweist, die sich von der magnetischen Suszeptibilität des anatomischen Gewebes unterscheidet, das die Anordnung umgibt, wenn sie in den menschlichen oder tierischen Körper eingebracht wird,
wobei jedes Muster (51, 52, 53, 54, 55) in einer vordefinierten räumlichen Beziehung zu einem der vordefinierten unterschiedlichen Punkte (2) steht, wobei die vordefinierten unterschiedlichen Punkte Elektrodenkontakte sind,
wobei
das Material ein Polymer ist, das insbesondere aus der Gruppe ausgewählt ist, umfassend Silikonkautschuk, Polyurethan, Polyimid, Epoxid, Flüssigkristallpolymer und Parylen,
**dadurch gekennzeichnet, dass**
das Material Teilchen mit einem Material umfasst, das aus der Gruppe ausgewählt ist, umfassend Eisen, Eisenoxid, Oxide von Seltenerden und Pyrolysekohlenstoffmaterial,
die Elektrodenkontakte ein erstes Gitter mit einer ersten Gitterkonstante ausbilden und die Muster (51, 52, 53, 54, 55) ein zweites Gitter mit derselben Gitterkonstante wie die der Elektrodenkontakte ausbilden.

2. Implantierbare Elektrodenanordnung nach Anspruch 1, wobei
die Muster (51, 52, 53, 54, 55) ein Material umfassen, das eine magnetische Suszeptibilität aufweist, die höher als die magnetische Suszeptibilität des anatomischen Gewebes ist, das die Elektrodenanordnung umgibt, wenn sie in den menschlichen oder tierischen Körper eingebracht wird.

3. Implantierbare Elektrodenanordnung nach einem der vorstehenden Ansprüche, wobei
jedes Muster (51, 52, 53, 54, 55) isoliert und von jedem anderen Muster (51, 52, 53, 54, 55) beabstandet ist.

4. Implantierbare Elektrodenanordnung nach einem der vorstehenden Ansprüche, wobei
die Struktur (5) ein Gitter über dem vordefinierten Abschnitt der Elektrodenanordnung ausbildet.

5. Implantierbare Elektrodenanordnung nach einem der vorstehenden Ansprüche, wobei
der vordefinierte Abschnitt mindestens eines umfasst von
- einer oberen Oberfläche (8),
- einer hinteren Oberfläche,
- einer Kante,
- einer Ecke.

6. Implantierbare Elektrodenanordnung nach einem der vorstehenden Ansprüche, wobei
mindestens drei Muster (51, 52, 53) entlang einer geraden ersten Linie ausgerichtet sind und mindestens drei Muster (52, 54, 55) entlang einer geraden zweiten Linie ausgerichtet sind, wobei die zweite Linie senkrecht zu der ersten Linie steht.

7. Implantierbare Elektrodenanordnung nach einem der vorstehenden Ansprüche, wobei
die implantierbare Elektrodenanordnung mindestens eine Schicht eines Polymers umfasst, das insbesondere aus der Gruppe ausgewählt ist, umfassend Silikonkautschuk, Polyurethan, Polyimid, Epoxid, Flüssigkristallpolymer und Parylen.

8. Verfahren zum Herstellen einer implantierbaren Elektrodenanordnung, die zum Einbringen in anatomisches Gewebe eines menschlichen oder tierischen Körpers geeignet ist, nach einem der vorstehenden Ansprüche, umfassend die folgenden Schritte:
a. eine erste Schicht eines Polymers wird auf einen mechanischen Träger aufgebracht;
b. die erste Polymerschicht wird gehärtet;
c. eine leitfähige Schicht wird auf die erste Polymerschicht aufgebracht;
d. die leitfähige Schicht wird mit einem Laser mit einem Muster versehen, wobei so Elektrodenkontakte (2), Verbindungsleiterbahnen und Schweißpads ausgebildet werden;
e. eine zweite Polymerschicht wird aufgebracht;
f. die zweite Polymerschicht wird gehärtet;
g. Gräben werden unter Verwendung eines Lasers in die Polymerschicht geschnitten, wobei die Gräben eine MRI-Referenzstruktur (5) definieren;
h. die Gräben werden mit einem Polymer gefüllt, umfassend ein Material, das eine magnetische Suszeptibilität aufweist, die sich von der magnetischen Suszeptibilität des anatomischen Gewebes unterscheidet, das die Elektrodenanordnung umgibt, wenn sie in den menschlichen oder tierischen Körper eingebracht wird; wobei das Polymer insbesondere aus der Gruppe ausgewählt ist, umfassend Silikonkautschuk, Polyurethan, Polyimid, Epoxid, Flüssigkristallpolymer und Parylen; das Polymer umfassend Partikel mit einem Material, das aus der Gruppe ausgewählt ist, umfassend Eisen, Eisenoxid, Oxide von Seltenerden und Pyrolysekohlenstoffmaterial;
i. die Füllung der Gräben wird gehärtet;
j. eine Deckschicht aus Polymer wird aufgebracht und gehärtet;
k. elektrische Kontakte werden durch Entfernen der Deckschicht mit einem Laser freigelegt;
l. die Kontur der Elektrodenanordnung wird durch Schneiden durch alle aufgebrachten Schichten mit einem Laser definiert;
m. die Elektrodenanordnung wird von dem mechanischen Träger getrennt.

9. Verfahren nach dem vorstehenden Anspruch, wobei
das Polymer aus der Gruppe ausgewählt ist, umfassend Silikonkautschuk, Polyurethan, Polyimid, Epoxid, Flüssigkristallpolymer und Parylen.

10. Verfahren nach dem vorstehenden Anspruch, wobei die leitfähige Schicht aus der Gruppe ausgewählt ist, umfassend Metall, leitfähiges Polymer, Kohlenstoff.

11. Verfahren nach einem der Ansprüche 9 und 10, wobei das Material eine magnetische Suszeptibilität aufweist, die höher als die magnetische Suszeptibilität des anatomischen Gewebes ist, das die Elektrodenanordnung umgibt, wenn sie in den menschlichen oder tierischen Körper eingebracht wird.

## Revendications

1. Réseau d'électrodes implantable apte à être placé dans un tissu anatomique d'un corps humain ou animal, comprenant
une structure (5) permettant de référencer des points distincts (2) prédéfinis du réseau d'électrodes implantable dans des images par résonance magnétique, la structure (5) étant agencée dans une partie prédéfinie du réseau d'électrodes implantable, la structure (5) comprenant :
une pluralité de motifs (51, 52, 53, 54, 55), chaque motif (51, 52, 53, 54, 55) ayant une forme prédéfinie et comprenant un matériau ayant une susceptibilité magnétique qui est différente de la susceptibilité magnétique du tissu anatomique entourant le réseau lorsqu'il est placé dans le corps humain ou animal,
chaque motif (51, 52, 53, 54, 55) étant dans une relation spatiale prédéfinie avec l'un des points distincts (2) prédéfinis, les points distincts prédéfinis étant des contacts d'électrodes,
dans lequel
le matériau est un polymère, en particulier choisi dans le groupe comprenant le caoutchouc de silicone, polyuréthane, polyimide, époxy, polymère à cristaux liquides et parylène,
**caractérisé en ce que**
le matériau comprend des particules comportant un matériau choisi dans le groupe comprenant le fer, oxyde de fer, oxydes de terres rares et matériau de carbone pyrolytique,
les contacts d'électrodes forment une première grille présentant une première constante de grille, et les motifs (51, 52, 53, 54, 55) forment une seconde grille présentant la même constante de grille que celle des contacts d'électrodes.

2. Réseau d'électrodes implantable selon la revendication 1, dans lequel
les modèles (51, 52, 53, 54, 55) comprennent un matériau ayant une sensibilité magnétique qui est supérieure à la sensibilité magnétique du tissu anatomique entourant le réseau d'électrodes lorsqu'il est placé dans le corps humain ou animal.

3. Réseau d'électrodes implantable selon l'une des revendications précédentes, dans lequel
chaque motif (51, 52, 53, 54, 55) est isolé et espacé de chaque autre motif (51, 52, 53, 54, 55).

4. Réseau d'électrodes implantable selon l'une des revendications précédentes, dans lequel
la structure (5) forme une grille sur la partie prédéfinie du réseau d'électrodes.

5. Réseau d'électrodes implantable selon l'une des revendications précédentes, dans lequel
la partie prédéfinie comprend au moins l'un parmi
- une surface supérieure (8),
- une surface arrière,
- un bord,
- un coin.

6. Réseau d'électrodes implantable selon l'une des revendications précédentes, dans lequel
au moins trois motifs (51, 52, 53) sont alignés le long d'une première ligne droite, et au moins trois motifs (52, 54, 55) sont alignés le long d'une seconde ligne droite, la seconde ligne étant perpendiculaire à la première ligne.

7. Réseau d'électrodes implantable selon l'une des revendications précédentes, dans lequel
le réseau d'électrodes implantable comprend au moins une couche d'un polymère, en particulier choisi dans le groupe comprenant le caoutchouc de silicone, polyuréthane, polyimide, époxy, polymère à cristaux liquides et parylène.

8. Procédé permettant la fabrication d'un réseau d'électrodes implantable, apte à être placé dans un tissu anatomique d'un corps humain ou animal, selon l'une des revendications précédentes, comprenant les étapes suivantes :
a. une première couche d'un polymère est déposée sur un support mécanique ;
b. la première couche de polymère est durcie ;
c. une couche conductrice est déposée sur la première couche polymère ;
d. la couche conductrice est façonnée à l'aide d'un laser, formant ainsi des contacts d'électrodes (2), des pistes d'interconnexion et des tapis de soudure ;
e. une seconde couche de polymère est déposée ;
f. la seconde couche de polymère est durcie ;
g. des tranchées sont découpées dans la couche de polymère à l'aide d'un laser, les tranchées définissant une structure de référence IRM (5) ;
h. les tranchées sont remplies d'un polymère comprenant un matériau ayant une susceptibilité magnétique qui est différente de la susceptibilité magnétique du tissu anatomique entourant le réseau d'électrodes lorsqu'il est placé dans le corps humain ou animal ; le polymère étant en particulier choisi dans le groupe comprenant le caoutchouc de silicone, polyuréthane, polyimide, époxy, polymère à cristaux liquides et parylène ; le polymère comprenant des particules comportant un matériau choisi dans le groupe comprenant le fer, oxyde de fer, oxydes de terres rares et matériau carboné pyrolytique ;
i. le remplissage des tranchées est durci ;
j. une couche de recouvrement en polymère est déposée et durcie ;
k. des contacts électriques sont exposés en retirant la couche de recouvrement à l'aide d'un laser ;
l. le contour du réseau d'électrodes est défini en découpant toutes les couches déposées à l'aide d'un laser ;
m. le réseau d'électrodes est séparé du support mécanique.

9. Procédé selon la revendication précédente, dans lequel
le polymère est choisi dans le groupe comprenant le caoutchouc de silicone, polyuréthane, polyimide, époxy, polymère à cristaux liquides et parylène.

10. Procédé selon la revendication précédente, dans lequel la couche conductrice est choisie dans le groupe comprenant un métal, polymère conducteur, carbone.

11. Procédé selon l'une des revendications 9 et 10, dans lequel le matériau a une susceptibilité magnétique qui est supérieure à la susceptibilité magnétique du tissu anatomique entourant le réseau d'électrodes lorsqu'il est placé dans le corps humain ou animal.
